(19) Europäisches Patentamt
European Patent Office
Office européen des brevets

(11) EP 2 122 596 B1

(12) **FASCICULE DE BREVET EUROPEEN**

(45) Date de publication et mention
de la délivrance du brevet:
**09.01.2013 Bulletin 2013/02**

(21) Numéro de dépôt: **08775717.5**

(22) Date de dépôt: **12.03.2008**

(51) Int Cl.:
*G08B 21/02* (2006.01)    *A61B 5/024* (2006.01)
*A61B 5/11* (2006.01)

(86) Numéro de dépôt international:
**PCT/FR2008/050420**

(87) Numéro de publication internationale:
**WO 2008/132359 (06.11.2008 Gazette 2008/45)**

(54) **DISPOSITIF, SYSTEME ET PROCEDE DE TELEVIGILANCE AMBULATOIRE COMPORTANT UN DISPOSITIF DE DEBRUITAGE DU POULS**

AMBULATE FERNÜBERWACHUNGSVORRICHTUNG, SYSTEM UND VERFAHREN MIT GERÄUSCHUNTERDRÜCKUNG DES PULSES

AMBULATORY REMOTE VIGILANCE DEVICE, SYSTEM AND METHOD WITH PULSE DENOISING

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **12.03.2007 FR 0753768**

(43) Date de publication de la demande:
**25.11.2009 Bulletin 2009/48**

(73) Titulaire: **Institut National des Telecommunications (INT)
Groupe des Ecoles des Telecommunications (GET)
91011 Evry Cedex (FR)**

(72) Inventeur: **BALDINGER, Jean-Louis
F-91230 Montgeron (FR)**

(74) Mandataire: **Tanty, François
Cabinet Nony
3, rue de Penthièvre
75008 Paris (FR)**

(56) Documents cités:
**EP-A- 0 319 160      EP-A- 0 845 241
EP-A- 1 302 156      EP-A- 1 374 763
EP-A- 1 575 010      WO-A-97/14357
US-A- 4 258 719      US-A- 5 737 439**

**Description**

La présente invention concerne un procédé et un dispositif pour débruiter un capteur de pouls.

En raison du vieillissement de la population en Europe, la télémédecine, c'est-à-dire la médecine pratiquée à distance, connaît un développement croissant pour répondre à l'encombrement des hôpitaux et au désir des personnes âgées ou malades de rester chez elles. Le concept de télésurveillance à domicile a été développé afin que les patients puissent continuer à vivre chez eux tout en étant l'objet d'une surveillance médicale.

La demande FR 2 829 862 divulgue un dispositif pour détecter la chute d'une personne et la présence de signaux vitaux de celle-ci.

Le brevet US 7 175 601 divulgue un dispositif de mesure du pouls comportant un détecteur optique d'un faisceau ayant traversé des tissus associé à un accéléromètre. L'analyse du signal optique capté en regard du signal délivré par l'accéléromètre permet d'éliminer du signal de pouls les artefacts dus aux mouvements de la personne portant le dispositif.

La demande EP 1 297 784 divulgue un dispositif de mesure du pouls comportant deux capteurs de pouls.

La demande internationale WO91/11956 divulgue un dispositif de mesure du pouls comportant un capteur de type photopléthysmographique comportant une source lumineuse et un composant sensible à la lumière émise par la source et un système de débruitage, comportant un étage de filtrage pour éliminer les artefacts rapides et un étage de filtrage en peigne, configuré pour éliminer les fréquences multiples du fondamental de l'alimentation électrique.

Des dispositifs multicapteurs permettant de mesurer différents paramètres physiologiques sont par ailleurs connus. Dans de tels dispositifs, des capteurs de pouls sont utilisés et sont par exemple placés au niveau du coeur ou de la ceinture.

Lors de la détermination des mesures du pouls, les artefacts, signaux parasitaires ou bruit, peuvent être par erreur assimilés à des battements. Un critère prépondérant à considérer est la valeur du seuil de discrimination battement/artefact, c'est-à-dire le seuil temporel à partir duquel un signal reçu est considéré comme un signal utile (battement) où comme du bruit (artefact). A une fréquence de pouls maximale de 200 battements par minute (bpm), admise pour la population adulte ciblée, l'intervalle minimum entre deux battements est de 0,3 seconde. On pourrait ainsi choisir cette durée comme seuil de discrimination battement/artefact. Néanmoins, une telle valeur de 0,3 seconde ne permet pas une suppression totale des fronts d'artefacts rapides pour les fréquences de pouls inférieures à 200 bpm, en particulier les fréquences normales de pouls comprises entre 50 et 120 bpm.

Par ailleurs, l'acquisition brute du pouls ambulatoire, lorsque le patient se déplace, entraîne une dispersion importante des mesures. Cette dispersion des mesures rend plus complexe leur exploitation et affecte également leur fiabilité. Cette dispersion s'explique entre autres par l'effet de bord apparaissant aux extrémités d'intervalles de temps perturbés de façon continue par les artefacts engendrés par les mouvements divers du patient. La discrimination battement/artefact peut s'avérer difficile par une méthode approchée.

Il existe donc un besoin pour bénéficier d'un dispositif relativement fiable, léger, peu coûteux, tout en étant performant, permettant notamment de réduire la dispersion des mesures de pouls acquises en ambulatoire.

L'invention vise notamment à répondre à ce besoin.

Selon l'un de ses aspects, l'invention a pour objet un dispositif de mesure du pouls tel que défini à la revendication 1. Selon l'un des aspects, le dispositif comporte :

- un capteur de pouls de type photopléthysmographique, de préférence porté à l'oreille, comportant au moins une source lumineuse, notamment une source de lumière infrarouge, et un composant sensible à la lumière émise par la source, et
- un système de débruitage comportant :

  - un circuit électronique de préconditionnement agencé pour éliminer les artefacts lents d'un signal représentatif du pouls acquis par le composant sensible à la lumière, et
  - au moins un microcontrôleur agencé pour traiter le signal délivré par le circuit électronique de préconditionnement et éliminer les artefacts rapides.

Par « artefact lent », on désigne tout artefact dont le spectre ne comporte pas de composante à une fréquence supérieure à 0,5 Hz.

Par « artefact rapide », on désigne tout artefact dont le spectre ne comporte pas de composante à une fréquence inférieure à 0,5 Hz.

Par « ambulatoire » ou « en ambulatoire », on désigne toute situation au cours de laquelle le patient présente une activité physique correspondant à un déplacement de ce dernier ou à un mouvement de son corps.

Un tel débruitage résultant d'un prétraitement électronique du signal acquis par le capteur de pouls ainsi que d'un post-traitement algorithmique effectué par le microcontrôleur permet de réduire d'un facteur 10 par exemple la dispersion des mesures de pouls acquises en ambulatoire par rapport à une acquisition brute traditionnelle. La marge d'erreur sur les mesures ambulatoires peut être, grâce à l'invention, réduite à 5 % par exemple.

Le dispositif peut ne comporter qu'un seul capteur de pouls.

Le dispositif peut ne comporter qu'une seule source lumineuse et qu'seul composant sensible à la lumière émise par la source lumineuse.

Le microcontrôleur et le circuit de préconditionnement étant par exemple situés dans un même boîtier pouvant être porté par le patient, l'invention permet de bénéficier d'un dispositif compact et facile à utiliser.

L'invention peut permettre une fiabilisation de l'acquisition du pouls en ambulatoire, c'est-à-dire une meilleure prise en compte, lors du traitement, des phénomènes d'incertitude.

La source lumineuse peut comporter une ou plusieurs diodes électroluminescentes (LED) générant un faisceau infrarouge, par exemple à une longueur d'onde de 950 nm, à travers un tissu, de préférence le lobe de l'oreille du patient.

Lorsque la source lumineuse comporte plusieurs LEDs, ces dernières peuvent ne constituer qu'une seule source lumineuse et n'être alimentées que par un unique générateur.

La ou les LEDs peuvent être alimentées de façon discontinue. L'extinction de la ou des LEDs en raison de l'alimentation discontinue peut permettre de supprimer les bruits additifs.

Par « bruit additif », on désigne les composantes du bruit à variations lentes, par exemple causées par un changement de position de la tête du patient par rapport à une source principale de lumière ambiante.

Le courant d'alimentation peut être un courant rectangulaire. Le rapport cyclique de ce courant d'alimentation est avantageusement inférieur à 1/10, étant par exemple compris entre 1/40 et 1/10, par exemple égal à 1/20. Le choix d'un tel rapport cyclique peut permettre de réduire le courant consommé pour l'alimentation de la ou des LEDs, ce qui peut permettre de bénéficier d'un dispositif à grande autonomie. Le dispositif selon l'invention peut par exemple présenter une autonomie d'au moins un mois d'utilisation continue sans recharge de la batterie ou échange des piles.

Afin de réduire encore la consommation du dispositif, l'alimentation d'un amplificateur connecté au composant sensible à la lumière peut également être interrompue en début de phase d'extinction de la ou des LEDs et rétablie peu avant l'allumage de la ou des LEDs.

En raison de sa grande autonomie et de la faible dispersion des mesures de pouls, le dispositif est particulièrement adapté à une mesure du pouls en ambulatoire.

L'alimentation de la ou des LEDs de façon discontinue peut encore permettre d'accroître le rapport signal sur bruit du signal en sortie du capteur de pouls de type photopléthysmographique.

Le composant sensible à la lumière peut comporter un phototransistor agencé pour capter le faisceau infrarouge ayant traversé le lobe de l'oreille du patient.

En variante, le faisceau infrarouge ayant traversé le lobe de l'oreille peut être capté par une photodiode ou tout autre composant sensible à la lumière.

Le circuit électronique peut comporter une chaîne de filtrage du signal capté. La chaîne de filtrage peut comporter un étage agencé pour soustraire du signal capté les bruits additifs.

La chaîne de filtrage peut comporter un étage de filtrage en peigne, agencé pour filtrer les fréquences multiples de la fréquence du fondamental de l'alimentation électrique de la source lumineuse. Dans le cas d'une alimentation à 50 Hz, cet étage est agencé pour filtrer les fréquences multiples de 100 Hz. Cet étage comporte par exemple deux voies d'acquisition comportant chacune un échantillonneur-bloqueur, chacun de ces échantillonneur-bloqueurs étant commandé en alternance.

La chaîne de filtrage peut comporter au moins un filtre agencé pour couper les fréquences à l'extérieur d'une fenêtre spectrale comprise entre 0,1 Hz et 20 Hz, mieux entre 0,5 Hz et 10 Hz.

Le circuit électronique de préconditionnement est de préférence entièrement analogique et peut être dépourvu de convertisseur analogique/numérique.

Un circuit de préconditionnement entièrement analogique peut permettre, en cas de numérisation ultérieure du signal d'optimiser le rapport signal de pouls sur bruit de quantification. Le circuit électronique de préconditionnement peut comporter un comparateur de tension à hystérésis, notamment flottant, comparant le signal issu de la chaîne de filtrage et une version retardée de ce même signal. Le retard peut être compris entre 20 ms et 40 ms.

Le dispositif de mesure du pouls peut ne comporter qu'un seul circuit électronique de préconditionnement.

Le signal est par exemple mis en forme rectangulaire à l'issue du circuit électronique de préconditionnement.

Le microcontrôleur peut exécuter un algorithme de débruitage.

L'invention a également pour objet, selon un autre de ses aspects, un système de surveillance mobile d'un patient tel que défini à la revendication 8.

Selon un aspect, le système de surveillance mobile comporte :

- un terminal multicapteur comportant un boîtier à porter par le patient, de préférence à la ceinture, le terminal incluant le dispositif de débruitage de la mesure du pouls tel que défini précédemment, et
- une base locale de traitement, par exemple de type PC, pour recevoir et traiter les informations envoyées par le terminal multicapteur. Cette base locale se trouve par exemple dans le lieu d'habitation du patient ou dans un local technique lorsque le système de surveillance mobile selon l'invention est déployé dans une maison de retraite médicalisée ou non, ou encore dans un centre de soins.

Le terminal multicapteur peut envoyer les informations à la base locale selon une périodicité prédéfinie, notamment selon une périodicité de trente secondes, voire de quinze secondes.

Le système peut comporter des moyens de fixation du boîtier à la ceinture, par exemple une boucle, un crochet, une fixation de type Velcro® ou tout autre moyen approprié.

La base locale peut être agencée pour traiter et fusionner les informations envoyées par le terminal multicapteur en vue de produire éventuellement une alarme

La base locale peut être agencée pour transmettre à un centre médical ou à un cabinet d'infirmières ou à un voisin prédéfini un message d'avertissement lorsque le capteur de pouls n'est pas porté par le patient. Dans le cas où le capteur comporte une pince ou tout autre moyen de fixation au niveau du lobe de l'oreille, un tel message d'avertissement peut par exemple être transmis lorsque le moyen de fixation n'est pas porté.

De même, un message d'avertissement peut être transmis par la base locale si le terminal multicapteur n'est pas porté à la ceinture grâce par exemple à un micro interrupteur au contact duquel vient le boîtier du terminal lorsqu'il est fixé à la ceinture.

Un tel dispositif permet par exemple de détecter si le boîtier du terminal a été déposé par le patient.

La base locale peut également être agencée pour transmettre un message d'avertissement lorsque le capteur de pouls n'est pas connecté au terminal multicapteur, par exemple si un fil de connexion est coupé ou bien en cas de déconnexion volontaire ou non.

Le terminal multicapteur peut comporter au moins un capteur d'actimétrie choisi dans la liste suivante : capteur de mouvement isotrope, capteur d'inclinaison et capteur d'impact de chute.

Le terminal multicapteur peut comporter un microcontrôleur agencé pour interpréter et débruiter les informations venant d'au moins un capteur d'actimétrie.

L'invention peut permettre d'obtenir au cours d'une durée donnée des mesures relatives au pouls et à l'actimétrie du patient, comme par exemple l'orientation de son corps par rapport à un axe horizontal et un axe vertical.

Le capteur d'impact de chute peut comporter quatre branches et chaque branche comporter en série un détecteur d'inclinaison et un capteur d'accélération.

Le terminal multicapteur peut comporter un bouton d'appel d'urgence agencé pour déclencher l'envoi d'informations vers la base locale. Ces informations peuvent être les données les plus récentes, ayant par exemple été acquises depuis l'envoi d'informations précédent.

Le terminal multicapteur peut comporter un commutateur marche/arrêt comportant deux sections, le commutateur étant agencé pour ne couper l'alimentation du terminal qu'après l'envoi immédiat à la base locale des données les plus récentes, accompagné d'un indicateur de mise sur arrêt du terminal multicapteur. A la suite de cet envoi immédiat, un dernier envoi périodique des données avant arrêt effectif peut encore avoir lieu, répétant dans les données transmises l'indicateur de mise sur arrêt du terminal multicapteur.

L'invention a encore, selon un autre de ses aspects, pour objet un procédé tel que défini à la revendication 12 pour mesurer le pouls d'une personne au moyen d'un dispositif de mesure du pouls.

Selon un aspect, le dispositif de mesure de pouls comporte :

- un capteur de pouls, de préférence porté à l'oreille, comportant au moins une source lumineuse et un composant sensible à la lumière émise par la source, et
- un système de débruitage comportant :

  - un circuit électronique de préconditionnement agencé pour éliminer les artefacts lents d'un signal représentatif du pouls acquis par le composant sensible à la lumière, et
  - au moins un microcontrôleur agencé pour traiter le signal délivré par le circuit électronique de préconditionnement et éliminer les artefacts rapides.

Le pouls de la personne peut être mesuré en ambulatoire.

Le procédé peut comporter une étape de prétraitement par le circuit électronique de préconditionnement au cours de laquelle :

- le signal issu du capteur de pouls attaque un étage de soustraction des composantes additives du bruit à variations lentes contenues dans le signal,
- le signal est ensuite traité par un étage de filtrage, notamment un étage de filtrage passe-bas à temps de propagation constant et à suppression des fréquences multiples du fondamental de l'alimentation électrique, et
- le signal est mis sous forme logique au moyen d'un étage comparateur.

Le procédé peut comporter une étape de post-traitement algorithmique par le microcontrôleur, au cours de laquelle :

- une suppression de l'effet de bord est effectuée en soustrayant du nombre de battements mesurés par intervalle de temps un nombre prédéfini de battements par zone bruitée, notamment un demi-battement par zone bruitée,
- on ajoute à la valeur obtenue le nombre de battements corrigés lors de l'intervalle de temps précédent *prorata-temporis* du temps bruité,
- on compare le taux temporel de bruitage de l'intervalle considéré à une valeur de référence, et
- on adapte le seuil de discrimination battement/artefact.

On désigne par « temps bruité » ou « taux temporel de bruitage » le taux temporel d'artefact compté pendant un intervalle de temps donné, par exemple quinze secondes.

Le seuil de discrimination battement/artefact peut par exemple être estimé de façon récursive.

La valeur de référence du taux temporel de bruitage est par exemple comprise entre 10 et 30 %, étant par exemple égale à 17 %.

En cas de taux temporel de bruitage supérieur à la valeur de référence, un message d'échec de débruitage peut être émis.

Selon un autre de ses aspects, on décrit un procédé pour mesurer le pouls d'une personne au moyen d'un dispositif de mesure du pouls comportant :

- un capteur de pouls, de préférence porté à l'oreille, comportant au moins une source lumineuse et un composant sensible à la lumière émise par la source et,
- un système de débruitage comportant :

    - un circuit électronique de préconditionnement agencé pour éliminer les artefacts lents d'un signal représentatif du pouls acquis par le composant sensible à la lumière,
    - au moins un microcontrôleur agencé pour traiter le signal délivré par le circuit électronique de préconditionnement et diminuer les artefacts rapides,
    - une base locale agencée pour substituer une valeur d'alarme prédéfinie à la valeur de pouls mesurée en cas de disfonctionnement du capteur de pouls et/ou lorsque celui-ci n'est pas porté par le patient et pour envoyer un message d'alarme.

L'invention a encore pour objet, en combinaison avec ce qui précède, un capteur d'impact de chute intégré dans un terminal multicapteur fixé à la ceinture d'un patient, le terminal multicapteur comportant un microcontrôleur agencé pour interpréter et débruiter les informations venant du capteur d'impact de chute, le capteur d'impact de chute comportant au moins trois branches, par exemple quatre branches, chaque branche comportant un détecteur d'inclinaison incliné par rapport à l'axe du tronc du patient, par exemple positionné à 45° par rapport à l'axe du tronc du patient, et un capteur d'accélération positionné dans un plan normal à l'axe du tronc du patient, le détecteur d'inclinaison étant relié par l'une de ses bornes au capteur d'accélération.

Dans tout ce qui suit, on désigne par "axe du tronc du patient" l'axe qui est vertical lorsque le patient est debout.

Les projections des branches du capteur d'impact de chute dans un plan normal à l'axe du tronc du patient portant le terminal multicapteur peuvent former deux à deux des angles de 90° lorsque le capteur d'impact de chute comporte quatre branches ou des angles de 120° lorsque le capteur d'impact de chute comporte trois branches

Dans chaque branche, une des deux bornes du capteur d'accélération peut être connectée à une entrée en interruption du microcontrôleur, l'autre borne étant connectée à une première borne du détecteur d'inclinaison dont l'autre borne est connectée à la masse, ce qui permet que les capteurs d'accélération soient tous connectés à la même entrée en interruption du microcontrôleur. Les détecteurs d'inclinaison de chaque branche peuvent par ailleurs être directement connectés à quatre entrées de port distinctes du microcontrôleur.

Un tel capteur d'impact de chute présente par exemple une consommation électrique très réduite, voire nulle et permet de détecter une chute en position allongée de la personne sous la condition que cet impact soit enregistré dans la direction de la chute, ce qui peut constituer un préconditionnement intrinsèque au capteur de chute.

Le microcontrôleur peut exécuter un algorithme permettant de discerner la position debout ou assis de la position allongée, mais aussi de détecter les chutes du patient lors du passage de ce dernier de la position debout ou assis à la position allongée, ou encore lors d'une chute à partir de la position allongée, lorsque le patient est allongé sur un lit par exemple.

L'algorithme peut stocker en mémoire du microcontrôleur, l'état des détecteurs d'inclinaison pendant une durée prédéfinie, par exemple trois secondes, ce qui peut permettre de déterminer en cas d'interruption du microcontrôleur déclenchée par un impact au sol si le patient était debout ou assis pendant au moins une portion de la durée prédéfinie précédent l'interruption, par exemple pendant une seconde lorsque la durée prédéfinie est de trois secondes, ou à défaut, si le tronc du patient a pivoté d'au moins 180° durant la période prédéfinie précédent l'interruption.

Le microcontrôleur peut être agencé pour émettre une alarme de chute vers une base locale de traitement si le patient reste allongé durant un intervalle de temps prédéfini, par exemple trente secondes, consécutivement à un impact au sol pris en compte malgré d'éventuelles tentatives de relèvement avortées. En variante, l'intervalle de temps précité est par exemple de quatre-vingt-dix secondes.

L'invention pourra être mieux comprise à la lecture de la description détaillée qui va suivre, d'un exemple de mise en oeuvre de celle-ci, et à l'examen du dessin annexé, sur lequel :

- la figure 1 représente un exemple de dispositif selon l'invention,
- la figure 2 représente de façon schématique un exemple de terminal multiplicateur,
- la figure 3 est un schéma en blocs du dispositif de la figure 2,
- les figures 4, 5 et 6 représentent de façon schématique des parties du circuit électronique de préconditionnement,
- la figure 7 représente, sous forme schématique, des étapes effectuées lors de l'exécution de l'algorithme d'élimination des artefacts rapides,
- la figure 8 représente un schéma d'un procédé d'estimation récursive du seuil de discrimination battement/artefact selon l'invention,
- la figure 9 représente de façon schématique un exemple de base locale,
- la figure 10 représente de façon schématique le circuit électronique du commutateur marche/arrêt, et
- la figure 11 représente un exemple d'un capteur d'impact de chute selon l'invention.

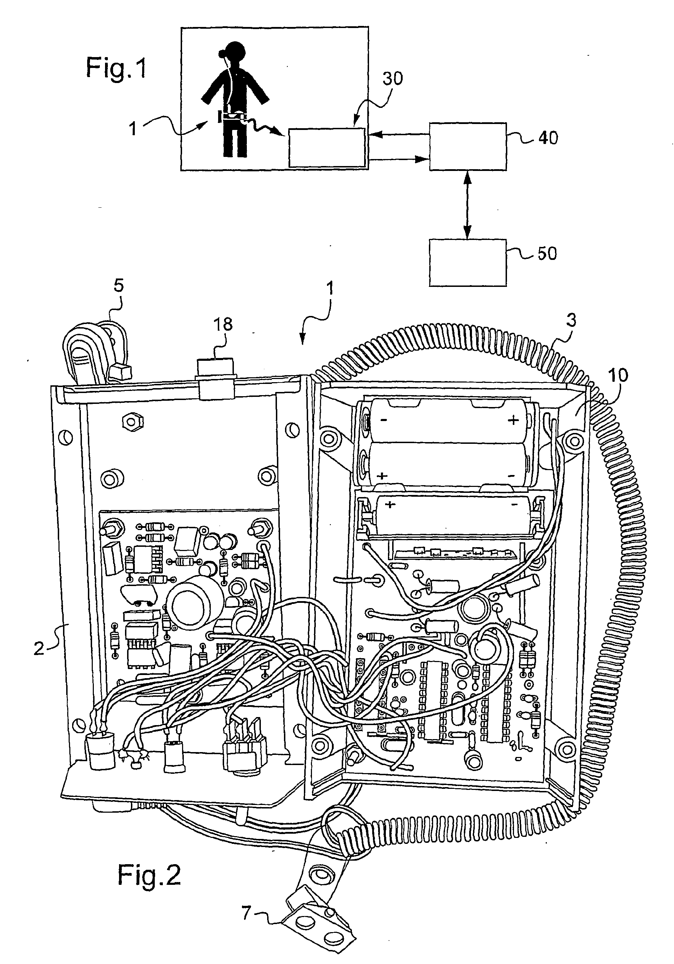

On a représenté à la figure 1 un exemple de dispositif conforme à l'invention.

Ce dispositif comporte un terminal multicapteur 1 et une base locale 30 pouvant communiquer avec un centre médical 40 ainsi qu'avec un médecin traitant 50, le cas échéant, ou encore avec un voisin prédéfini.

On a représenté à la figure 2 un exemple de réalisation du terminal multicapteur 1. Ce terminal multicapteur comporte un boîtier 2 fixé par exemple à la ceinture du patient par l'intermédiaire d'une pince 7 ou autrement.

Le terminal comporte un capteur photophléthysmographique de pouls 5 connecté au boîtier 2 par l'intermédiaire d'un câble 3. En variante, ce capteur 5 peut communiquer avec le boîtier 2 par l'intermédiaire d'une liaison radio-fréquence.

Le boîtier 2 comporte également un logement 10 destiné à recevoir des piles pour alimenter le système. En variante, l'alimentation peut être assurée par une batterie rechargeable. Un tel terminal multicapteur 1 peut présenter une consommation électrique très réduite, par exemple de 3mA.

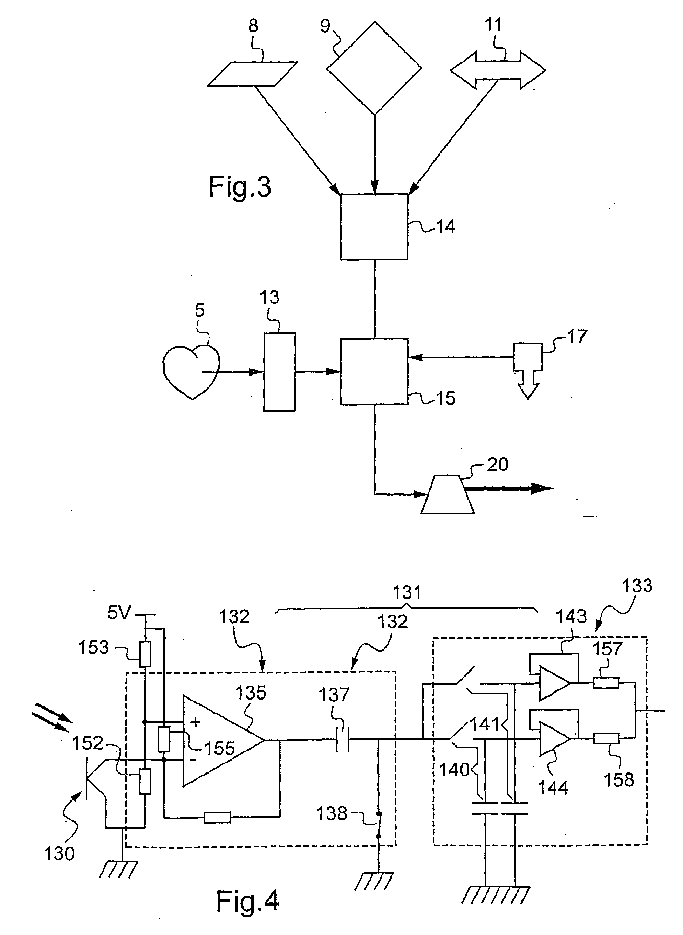

Le terminal multicapteur 1, représenté de façon schématique à la figure 3, peut comporter également, comme illustré, un capteur d'inclinaison 8, un capteur d'impact de chute 9, un capteur de mouvement 11, deux microcontrôleurs 14 et 15 ainsi qu'un bouton d'appel d'urgence 17.

Le boîtier 2 comporte encore un émetteur VHF 20 dont la portée radio est comprise entre quinze et vingt mètres en appartement et qui émet à une fréquence de 433 MHz, par exemple. En variante, il peut s'agir d'un émetteur UHF émettant à une fréquence de 2,4 GHz ou 868 MHz, par exemple.

Dans une autre variante, la liaison radio peut être bidirectionnelle.

Le capteur de pouls 5 utilisé dans l'exemple décrit est une pince d'oreille et peut comporter une ou plusieurs LEDs constituant une unique source lumineuse et émettant un faisceau infrarouge, par exemple à 950 nm, et un composant sensible à la lumière pouvant comporter un phototransistor ou, en variante, une ou plusieurs photodiodes infrarouges intégrées dans le même composant sensible à la lumière, permettant une mesure, par réfraction à travers le lobe de l'oreille, de l'atténuation du faisceau infrarouge issu de la ou des LEDs.

Le capteur 5 peut également comporter un dispositif optique adaptant la géométrie du faisceau infrarouge.

Le composant sensible à la lumière peut comporter un filtre infrarouge atténuant les perturbations dues à la lumière ambiante.

Ce capteur 5 peut être muni d'un crochet disposé derrière l'oreille ou peut, en variante, être logé dans les branches d'une monture de lunettes et peut comporter une ou plusieurs piles, être rechargé de façon inductive ou comporter une photopile.

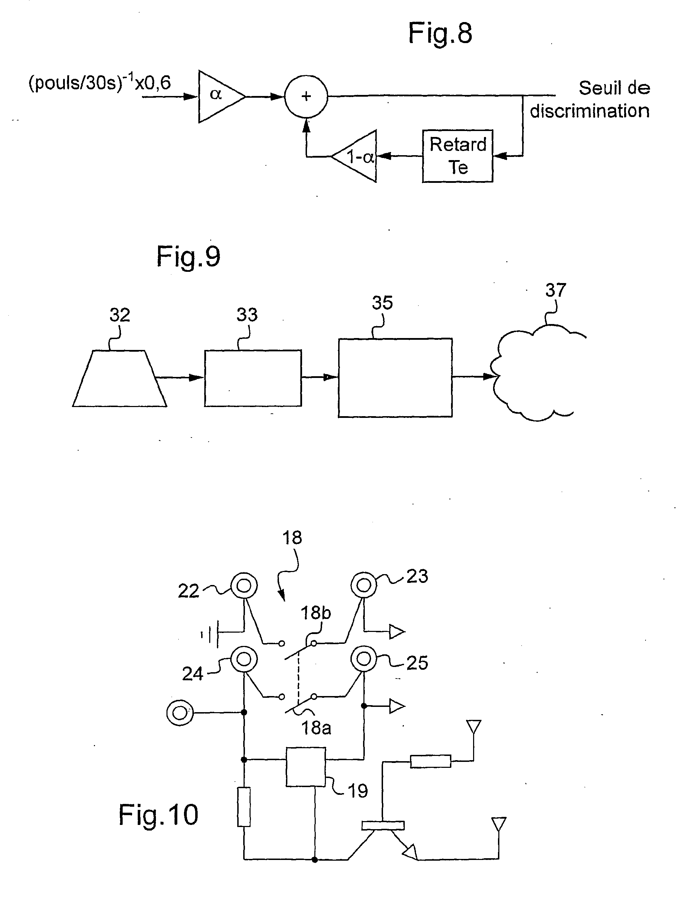

Le boîtier 2 peut comporter un commutateur marche/arrêt 18 qui est de type électromécanique et/ou à semi-conducteur. Dans un exemple de mise en oeuvre de l'invention, le commutateur 18, représenté à la figure 10, comporte deux interrupteurs 18a et 18b commandés ensemble mécaniquement. Le commutateur 18 comporte également un interrupteur électronique 19 qui est dans l'exemple décrit un transistor à effet de champ de type MOS, agencé de façon à « shunter » l'interrupteur 18a.

Le commutateur 18 peut ne pas être soudé sur la carte électronique du terminal multicapteur 1 et y être par exemple connecté par des fils souples à partir de points de connexion 22, 23, 24 et 25.

Le capteur d'inclinaison 8 comporte quatre capteurs de préférence à gouttes de mercure ou autre liquide conducteur. De tels capteurs sont moins perturbés par les mouvements du patient que des capteurs à bille. Les capteurs d'inclinaison 8 sont répartis selon quatre directions deux à deux orthogonales, les directions correspondant aux génératrices d'un cône pointant vers le bas, ouvert à 90° et dont l'axe coïncide avec celui du tronc du patient.

Le capteur de mouvement 11 comporte un capteur à bille, par exemple de la marque ASSEMtech, connecté à une entrée du microcontrôleur 14. Ce capteur de mouvement, après conditionnement par le microcontrôleur 14, permet de totaliser le nombre de secondes où le patient présente la moindre activité physique et peut permettre de connaître le taux temporel de mouvement du patient.

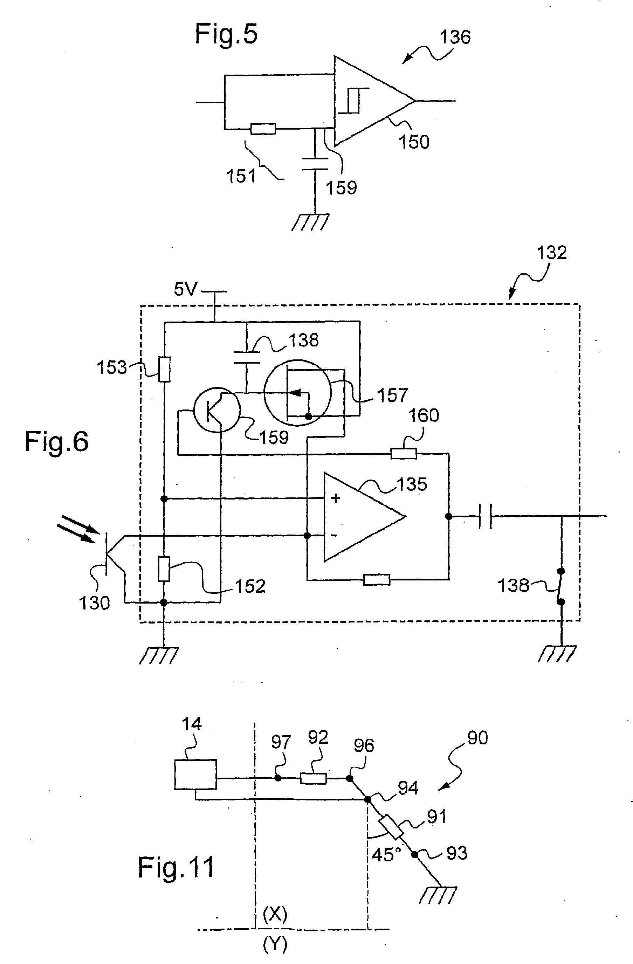

Le capteur d'impact de chute 9 est par exemple construit autour d'un capteur composite fournissant l'information de position (allongé ou debout/assis) et d'impact de chute au sol. Ce capteur comporte par exemple quatre branches dont on a représenté un exemple à la figure 11. Chaque branche 90 comporte un détecteur d'inclinaison 91 par rapport à l'horizontale, par exemple similaire au capteur d'inclinaison 8, et un capteur d'accélération 92, par exemple un interrupteur détecteur de dépassement d'un seuil d'accélération fixé par exemple à 2 g.

Les détecteurs d'inclinaison 91 sont dans l'exemple décrit positionnés à 45° par rapport à l'axe X du tronc du patient portant le terminal multicapteur 1 et les capteurs d'accélération 92 peuvent être positionnés dans un plan normal Y à l'axe X du tronc du patient. Les interrupteurs détecteurs de dépassement du seuil d'accélération 92 sont par exemple agencés pour se fermer brièvement lorsque la projection de l'accélération sur leur axe dépasse le seuil d'accélération fixé.

Dans chaque branche 90, le détecteur d'inclinaison 91 comporte deux bornes 93 et 94. La première borne 93 est connectée à la masse, la deuxième borne 94 est connectée à une première borne 96 du capteur d'accélération 92 de la même branche. La deuxième borne 97 des capteurs d'accélération de chaque branche est connectée à la même entrée en interruption du microcontrôleur 14. Par ailleurs, le détecteur d'inclinaison 91 de chaque branche 90 est directement connecté par sa deuxième borne 94 à des entrées de port distinctes du microcontrôleur 14.

On obtient ainsi, dans cet exemple, un capteur d'impact à quatre branches orthogonales procédant par filtrage d'orientation, chacune de ces branches étant composée d'un détecteur d'inclinaison et d'un capteur d'accélération en série. On parle de filtrage d'orientation puisque ce dispositif n'enregistre un impact de chute que si un impact est détecté en position allongée dans la direction où le patient s'est allongé.

Le microcontrôleur 14 est agencé pour interpréter et débruiter les informations venant des capteurs d'inclinaison 8 et de mouvement 11.

Le microcontrôleur 14 est agencé pour traiter les informations reçues du capteur d'impact de chute 9, pour associer à une variable une valeur lorsqu'une information de chute est détectée, et pour émettre ensuite une alarme de chute si la variable conserve la même valeur durant une durée prédéfinie, de préférence comprise entre dix et cent secondes, par exemple de trente secondes, c'est-à-dire si la personne équipée reste continuellement allongée pendant cette durée.

Le microcontrôleur 14 est par exemple agencé pour discerner la position debout ou assis de la position allongée ainsi que pour détecter d'éventuelles chutes du patient, aussi bien depuis une position assise ou debout à une position allongée que lors d'une chute depuis une position allongée, ce qui est par exemple le cas lorsqu'un patient tombe du lit dans lequel il est allongé.

Dans l'exemple décrit, le microcontrôleur 14 est agencé pour stocker dans sa mémoire l'état des détecteurs d'inclinaison pendant une durée prédéfinie, étant dans l'exemple décrit égale à trois secondes, pour déterminer en cas d'une interruption du microcontrôleur 14 déclenchée par un impact au sol si, durant la durée prédéfinie précédant l'interruption, le patient était debout ou assis ou si le tronc du patient en position allongée a pivoté de plus de 180°.

Dans un exemple de mise en oeuvre de l'invention, lorsqu'un patient ayant subi une chute essaie vainement de se relever, les mesures acquises après la chute qui ne traduisent plus une position allongée ne doivent pas modifier la valeur de la variable associée à l'information de chute. Une telle détection par le microcontrôleur 14 du retour à la position debout/assis peut être insensible aux éventuelles tentatives vaines de relèvement du patient.

Dans ce but, si un impact de chute a été détecté par le capteur d'impact de chute 9, le microcontrôleur 14 peut être agencé pour, dans le cadre de la validation de l'alarme de chute, modifier la fréquence d'échantillonnage des mesures des capteurs d'actimétrie, notamment des capteurs d'inclinaison. Le microcontrôleur 14 peut échantillonner par exemple toutes les 50 ms l'état du capteur d'inclinaison 8 et détecter l'état debout/assis pour une fraction, par exemple 1/3, des échantillons acquis pendant la durée prédéfinie de validation de l'envoi de l'alarme dé chute. Lorsque la durée prédéfinie de validation de l'alarme de chute est de trente secondes, le microcontrôleur 14 peut détecter l'état debout/assis toutes les dix secondes pendant trois secondes.

La valeur de la variable associée à l'information de chute peut n'être pas modifiée, ce qui peut conduire à l'envoi de l'alarme de chute à l'issue de la durée prédéfinie.

Dans un autre exemple de mise en oeuvre de l'invention, la durée de validation avant émission de l'alarme peut être plus élevée.

Dans un autre exemple de mise en oeuvre de l'invention, le terminal multicapteur 1 comporte un buzzer émettant un premier signal audible de durée d'environ 100 ms toutes les trois secondes tant que l'alarme de chute n'a pas été validée puis un deuxième signal de durée d'environ 1 seconde lorsque l'alarme a été validée.

L'alarme de chute est émise dès qu'elle a été validée à la base locale 30 puis peut être réémise lors d'une émission ordinaire suivante de données

Le microcontrôleur 14 envoie périodiquement, par exemple toutes les quinze secondes, les informations débruitées au microcontrôleur 15.

Dans l'exemple considéré, le microcontrôleur 15 envoie par exemple toutes les trente secondes un signal sur plusieurs octets à l'émetteur 20. Le premier octet comporte par exemple des informations relatives à l'actimétrie mesurée par les capteurs d'inclinaison 8, d'impact de chute 9, de mouvement 11. Ces informations peuvent être relatives au niveau de charge de la batterie ou des piles du boîtier 2 ou encore à une action éventuelle sur le bouton d'appel d'urgence 17. Le deuxième octet correspond par exemple à une étiquette, le troisième octet comporte par exemple des informations sur le pouls, issues du procédé de débruitage et le quatrième octet correspond par exemple à la répétition de l'étiquette précédente, ce qui peut fournir un moyen de déceler une erreur de transmission dans les données reçues et de fiabiliser, dans le cas d'une utilisation en maison de retraite ou dans un centre de soins, la connaissance de la provenance des données pour une utilisation de plusieurs terminaux multicapteur 1 avec une seule base locale 30.

Le microcontrôleur 15 est également agencé dans l'exemple considéré pour superviser le fonctionnement du bouton d'appel d'urgence 17 dont l'actionnement peut déclencher l'envoi immédiat des informations des capteurs dont l'ancienneté n'excède pas par exemple quinze ou trente secondes et la répétition automatique de cette émission dans les trente secondes suivantes, par exemple.

Le microcontrôleur 15 peut être agencé pour superviser le capteur 5 et le circuit de préconditionnement 13 qui sera décrit par la suite. Le microcontrôleur 15 est par exemple agencé pour traiter le signal délivré par le circuit de préconditionnement 13, ce qui peut permettre d'atténuer relativement fortement les erreurs dues aux artefacts de mouvement du patient sur les mesures envoyées à la base locale 30 de réception du nombre de battements pendant un intervalle de temps par exemple égal à trente secondes.

La supervision mentionnée ci-dessus peut, en variante, être effectuée par le microcontrôleur 14.

Lorsque le patient décide de couper l'alimentation du boîtier 2, il peut exercer une action sur le commutateur 18 de façon à placer celui-ci sur la position « arrêt ».

Cette action entraîne l'ouverture de l'interrupteur 18b et l'envoi au microcontrôleur 15 d'une information relative à cette ouverture de l'interrupteur 18b.

Le microcontrôleur 15 peut être agencé pour maintenir le transistor MOS 19, utilisé en interrupteur et placé en parallèle sur l'interrupteur 18a, passant et provoquer l'envoi immédiat des informations courantes à la base locale 30.

Le microcontrôleur 15 peut maintenir le transistor MOS 19 passant tant que les informations transmises périodiquement à la base locale 30 n'ont pas été envoyées. Lorsque l'envoi périodique d'informations courantes à la base locale 30 a été effectué, le microcontrôleur 15 peut couper l'alimentation du boîtier en agissant sur la grille du transistor MOS 19.

L'envoi immédiat à la base locale d'informations et l'envoi d'informations périodiques peut permettre à la base locale de vérifier la vraisemblance des informations immédiatement envoyées, ce qui peut constituer une indication relative à la fiabilité de ces informations.

Dans une variante, le microcontrôleur 15 peut être agencé pour couper l'alimentation en agissant sur l'interrupteur 19 dès l'envoi immédiat d'informations à la base locale 30.

Un exemple de réalisation de la base locale 30 est représenté à la figure 9. La base locale 30 reçoit les informations envoyées par le terminal 1, notamment par l'émetteur 20. Elle comporte pour ce faire un récepteur 32, par exemple un récepteur VHF de fréquence par exemple égale à 433 MHz ou un récepteur UHF, par exemple à 2,4 GHz ou 868 MHz. Elle comporte également un microcontrôleur 33 gérant la réception des informations, un ordinateur ou système informatique 35 et une liaison, par exemple une liaison série RS 232, reliant le microcontrôleur 33 au port série de l'ordinateur 35. En variante, le microcontrôleur 33 et l'ordinateur 35 pourraient être connectés par une liaison parallèle ou une liaison USB, par exemple.

La base locale 30 est connectée par un réseau 37 pouvant être Internet ou tout autre réseau filaire ou encore un réseau non filaire, avec un serveur centralisé de télésurveillance situé dans le centre médical 40. Un médecin de garde 50 et/ou une infirmière du centre médical 40 peut émettre un prédiagnostic sur la base des informations lui ayant été transmises depuis la base locale 30 par le réseau 37 et de données personnelles du patient stockées dans une base de données du centre médical 40.

L'ordinateur 35 est, dans l'exemple décrit, agencé pour traiter les informations issues des capteurs, les fusionner à l'aide de règles de décision et enregistrer ces informations dans un fichier journalier puis un fichier archive ; une base d'archivage de profondeur ou nombre de jours adapté à l'exploitation du système, par exemple trente jours, peut par exemple être réalisée. L'ordinateur 35 comporte, dans l'exemple décrit, un écran permettant d'afficher les données ainsi traitées. Le PC 35 est également agencé pour ajouter deux valeurs de pouls calculées sur trente secondes, par exemple la n$^{ème}$ valeur de pouls, transmise par le microcontrôleur 15 du terminal multicapteur 1 à la n-1$^{ème}$ valeur de pouls afin de fournir une valeur en nombre de battements par minute. Le cas échéant, la base locale 30 peut émettre une alarme de chute, ou encore une alarme correspondant à l'action du patient sur le bouton d'appel d'urgence 17.

Les alarmes peuvent être transmises à des personnes sélectionnées par le patient comme un parent, un ami ou un voisin.

En réponse à un enlèvement du capteur 5 par le patient, la base locale 30 peut être agencée pour substituer une valeur d'alarme à la valeur de pouls, et pour provoquer l'affichage sur l'écran de l'ordinateur 35 et la transmission au centre médical 40 d'un message d'avertissement, par exemple « pince ôtée ». Cette valeur d'alarme peut être choisie de façon à ne pas pouvoir être interprétée comme une valeur plausible de battement. La fréquence de pouls maximale admise pour la population ciblée étant de 100 battements par intervalle de trente secondes, la valeur choisie sera par exemple supérieure à 110.

Le terminal multicapteur 1 peut être agencé pour détecter si la pince du capteur 5 est déconnectée du boîtier 2 par le patient ou si le fil d'alimentation des LEDs est coupé à l'intérieur du boîtier 2, ou encore dans le câble 3 du capteur de pouls, ou encore si la fiche du câble 3 est incorrectement engagée dans une prise du boîtier correspondante, pour substituer une valeur d'alarme choisie par exemple comme ci-dessus à la valeur du pouls et pour provoquer l'affichage sur l'écran de l'ordinateur 35 et la transmission au centre médical 40 d'un message d'avertissement, par exemple « pince déconnectée ».

Le dispositif peut par exemple permettre d'informer le centre médical 40 de l'état d'utilisation du boîtier et des pannes éventuelles du terminal multicapteur pour éviter, le cas échéant, le déplacement inutile et dommageable d'un véhicule sanitaire.

Le serveur centralisé de télésurveillance situé dans le centre médical 40 peut être agencé pour programmer la base locale 30 ou le terminal multicapteur 1. Le programme du serveur centralisé peut, comme déjà évoqué, permettre de déterminer si la pince capteur de pouls 5 est portée ou non, si la pince du capteur de pouls 5 est déconnectée du terminal multicapteur, si l'état de charge de la batterie ou des piles de l'alimentation du boîtier est suffisant ou non, ou encore si le terminal multicapteur a été arrêté de façon volontaire.

Le programme du serveur centralisé peut également être agencé pour contrôler le fonctionnement de la base locale 30 à partir de l'ordinateur 35 par renvoi d'un message spécifique en testant par exemple le fonctionnement normal du microcontrôleur 33 par envoi à ce microcontrôleur 33 d'un ordre de réinitialisation (*reset* du microcontrôleur) déclenchant en retour l'envoi vers l'ordinateur 35 d'un message spécifique.

On va maintenant décrire le dispositif de débruitage précédemment évoqué, mis en oeuvre dans le circuit électronique de préconditionnement 13 et le microcontrôleur 14 pour traiter les informations venant du capteur 5.

Ce capteur 5 comporte dans l'exemple décrit trois LEDs émettant un faisceau infrarouge à travers le lobe de l'oreille d'un utilisateur et constituant une unique source lumineuse. Dans le but de limiter la consommation des LEDs, celles-ci sont par exemple alimentées par l'intermédiaire du microcontrôleur 14 ou d'un microcontrôleur logé dans le capteur 5 qui délivre un courant rectangulaire à faible rapport cyclique, de préférence inférieur à 1/10, par exemple compris entre 1/40 et 1/10, par exemple égal à 1/20.

Le faisceau infrarouge éclaire un composant sensible à la lumière, dans l'exemple décrit un phototransistor 130. L'alimentation des LEDs est par exemple telle que le temps d'allumage de ces dernières soit de 500 $\mu$s.

L'invention n'est bien sûr pas limitée à l'emploi comme composant sensible à la lumière d'un phototransistor. Dans un autre exemple de mise en oeuvre de l'invention, le composant sensible à la lumière est une photodiode et l'alimentation des LEDs est telle que le temps d'allumage de ces dernières est de 50 $\mu$s.

La fréquence d'alimentation des LEDs peut être choisie pour satisfaire les contraintes d'autonomie du terminal multicapteur 1 et d'élimination de fréquences modulant les sources de lumière artificielle qui résultent généralement de la fréquence du secteur, soit 50 Hz et ses harmoniques par exemple. La fréquence d'alimentation des LEDs peut avantageusement être égale à 200 Hz.

Le signal issu du capteur 5 attaque ensuite le circuit de préconditionnement 13, qui est agencé pour éliminer les artefacts lents présents dans le signal du pouls capté par le capteur 5. Le circuit de préconditionnement 13 est partiellement représenté aux figures 4 et 5.

Ce circuit de préconditionnement comporte dans l'exemple décrit une chaîne de filtrage 131 comportant un étage 132 de soustraction des bruits additifs, un étage 133 de filtrage des fréquences multiples du fondamental de l'alimentation électrique, un filtre passe-haut, un gain variable, et un filtre passe-bas.

Le circuit de préconditionnement 13 comporte encore un étage 136 dit « trigger flottant » constitué d'un comparateur de tension à hystérésis.

Le signal capté par le phototransistor 130 et correspondant à l'éclairement du lobe du patient attaque le circuit 13 par l'intermédiaire de l'étage de soustraction 132 des bruits additifs.

Cet étage 132 est agencé pour soustraire les composantes du bruit à variations lentes, par exemple causées par un changement de position de la tête du patient par rapport à une source principale de lumière ambiante. De tels bruits parasitaires constituent généralement un obstacle à l'utilisation de terminaux multicapteurs en ambulatoire, étant engendrés par le moindre mouvement.

Le signal issu du phototransistor 130 attaque l'entrée - d'un amplificateur opérationnel (A.O.) 135. L'entrée + de l'A.O. 135 est alimentée par un pont diviseur de tension comportant deux résistances 152 et 153 et dont la tension d'alimentation est par exemple de 5 V. En choisissant des résistances 152 et 153 de même valeur et en alimentant le système par une tension de 5 V, l'entrée + de l'A.O. a pour potentiel 2,5 V et la tension de sortie de l'A.O. varie entre 2,5 et 5 V. Afin d'obtenir une variation de la tension de sortie de l'A.O. 135 comprise entre 0 et 5 V, une résistance 155 est montée en série entre l'alimentation (par exemple 5V) et l'entrée - de l'A.O. 135. Le signal en sortie de l'A.O. 135 attaque ensuite un ensemble comportant un condensateur 137 et un interrupteur électronique 138, par exemple un transistor à effet de champ ou un interrupteur analogique, par exemple commercialisé par la société MAXIM®.

Lorsque le phototransistor 130 est éclairé, l'interrupteur 138 est ouvert. La tension mesurée aux bornes de la capacité 137 correspond dans ce cas à l'éclairage issu des LEDs infrarouges et ayant par exemple traversé le lobe de l'oreille auquel s'ajoutent les bruits (variations lentes de la lumière ambiante...) indépendants de l'éclairage issu des LEDs.

Lorsque les LEDs infrarouges du capteur 5 sont éteintes, l'interrupteur 138 est fermé. La tension mesurée aux bornes du condensateur correspond dans ce cas aux bruits indépendants de l'éclairage par les LEDs.

Le cycle allumage-extinction des LEDs permet de soustraire la partie additive des variations lentes de l'éclairage au niveau du phototransistor, dues au mouvement de la tête du patient ainsi qu'aux modifications diverses de l'éclairage de son environnement proche, le condensateur 137 ayant à ses bornes une tension représentative de cette partie additive lorsque le phototransistor 130 n'est pas éclairé par les LEDs.

L'étage 133 permet d'éliminer du spectre de fréquence les multiples du fondamental de l'alimentation électrique.

L'éclairage ambiant est généralement alimenté à 50 Hz, ce qui correspond à un signal parasitaire comportant un fondamental à 100 Hz ainsi que plusieurs harmoniques, notamment à des fréquences multiples de 100 Hz. Dans le cas où la fréquence de l'alimentation électrique est de 60 Hz, le signal parasitaire comporte un fondamental à 120 Hz et des harmoniques à des multiples de 120 Hz.

L'étage 133 est agencé pour effectuer un filtrage à temps discret du signal issu de l'étage 132, sans qu'il y ait numérisation de ce signal. La complexité du dispositif est ainsi réduite.

Cet étage 133 comporte deux voies d'acquisition. Chaque voie comporte un échantillonneur-bloqueur 140 ou 141 monté en série avec un montage dit suiveur constitué d'un A.O. 143 ou 144. On ne sort pas du cadre de la présente invention lorsque l'étage 133 ne comporte qu'une voie d'acquisition et qu'un échantillonneur-bloqueur.

Les sorties de ces A.O. 143 et 144 sont respectivement connectées aux résistances 157 et 158.

Dans l'exemple décrit, ces deux résistances 157 et 158 sont de même valeur avec une tolérance par exemple égale à 0,1 %.

Une sommation des signaux provenant de chaque échantillonneur-bloqueur 140 et 141 est effectuée en sortie de l'étage 133.

Les échantillonneurs-bloqueurs 140 et 141 sont commandés alternativement, de façon à ce que le signal transite par une voie ou par l'autre, sans possibilité de recouvrement.

Durant les phases d'extinction des LEDs, le phototransistor 130 n'est pas éclairé. Les échantillonneur-bloqueurs 140 et 141, qui sont dans l'exemple décrit des bloqueurs d'ordre zéro permettent, lorsque les LEDs sont éteintes, le maintien du signal enregistré pendant l'éclairement du phototransistor par les LEDs.

La commande par alternance des échantillonneur-bloqueurs 140 et 141 permet que la $n^{\text{ème}}$ mesure ne soit acquise que par une voie, la $(n-1)^{\text{ème}}$ ayant été acquise par l'autre.

L'étage 133 effectue deux opérations de filtrage en cascade.

La première opération est un filtrage à temps échantillonné d'équation de récurrence

$$\frac{x(n) + x(n-1)}{2}$$

dont la réponse en fréquence, du type filtre en peigne, est

$$\cos\left(\frac{\omega T}{2}\right)$$

où $\omega$ est la pulsation du signal et T la période d'échantillonnage. Cette réponse s'annule aux demi-multiples impairs de la fréquence d'échantillonnage.

La deuxième opération est un filtrage à temps continu réalisé par la fonction « bloqueur d'ordre zéro » dont la réponse en fréquence est

$$\text{sinc} (\pi fT).$$

Cette réponse constitue un filtrage passe-bas dont la réponse en fréquence s'annule aux multiples de la fréquence d'échantillonnage.

La mise en cascade de ces deux opérations de filtrage peut permettre d'obtenir un filtrage dépourvu de distorsion de temps de propagation de groupe ou encore à propagation de groupe constant, ce qui peut permettre de retransmettre au mieux la forme du signal utile avant sa mise en forme par l'étage trigger flottant 136.

L'étage 133 étant agencé dans l'exemple considéré pour permettre l'élimination du spectre de fréquence des multiples de 100 Hz. La fréquence d'échantillonnage est choisie égale à 200 Hz, ce qui permet une commande alternée à 100 Hz des échantillonneur-bloqueurs 140 et 141.

Dans le cas où la fréquence de l'alimentation électrique est de 60 Hz, la fréquence d'échantillonnage est choisie égale à 240 Hz.

Le signal en sortie de l'étage 133 est dépourvu des bruits dont la fréquence est un multiple du fondamental (100 ou 120 Hz) de l'éclairage électrique alimenté par le secteur.

Le circuit de préconditionnement 13 comporte encore, dans l'exemple décrit, un filtre passe-haut de fréquence de coupure de 0,5 Hz, un amplificateur de gain variable et un filtre passe-bas de fréquence de coupure 10 Hz. Le signal issu de ces filtres est un signal lissé dans une fenêtre spectrale s'étendant par exemple entre 0,5 Hz et 20 Hz, ou encore entre 0,5 Hz et 10 Hz. La réduction de la fenêtre spectrale contenant le signal peut permettre par exemple d'accroître la valeur du rapport signal/bruit dans la mesure où les transitions rapides du signal (fronts systoliques) sont correctement retransmises par ce filtrage passe-bande de manière à ne pas perturber le fonctionnement de l'étage « trigger flottant » 136. Par « correctement retransmises », on entend avec une faible distorsion d'amplitude et de temps de propagation de groupe.

Le signal issu de l'étage 133 comporte les fronts rapides issus du capteur 5 mais les bruits à haute fréquence ont été supprimés.

Afin d'obtenir en sortie du circuit de préconditionnement 13 un signal rectangulaire, le signal attaque l'étage 136 dit « trigger flottant » représenté à la figure 5.

Cet étage 136 comporte un comparateur de tension à hystérésis 150 qui compare le signal issu de la chaîne de filtrage avec un signal en sortie d'un filtre 151. Ce filtre 151 est de type RC passe-bas utilisé comme élément de retard approximativement constant et présente à sa sortie 159 une version légèrement retardée du signal d'entrée.

Le signal issu de ce comparateur 136 est un signal logique présentant un front descendant sur chaque battement de pouls mais aussi en présence d'artefacts de mouvements rapides d'amplitude dépassant le seuil du comparateur 136, comme par exemple un choc sur le capteur ou un mouvement rapide de la tête.

On a représenté à la figure 6 un autre exemple d'étage 132 de soustraction des bruits additifs. Dans cet exemple, l'étage 132 diffère de celui décrit en référence à la figure 4 par le remplacement de la résistance 155 par un transistor MOSFET à canal P 157. La source de ce transistor 157 est alimentée par la tension d'alimentation qui est dans l'exemple décrit de 5V. Le drain du transistor est connecté à l'entrée - de l'A.O. 135. Un condensateur 138, de capacité par exemple comprise entre 2 nF et 10 nF, est connecté entre la grille et la source du transistor 157. Le condensateur 138 est dans l'exemple décrit agencé pour mémoriser la tension de commande correspondant à la fourniture d'un courant sensiblement égal mais opposé au photo-courant dû à l'éclairage ambiant, ce qui peut permettre d'accroître la dynamique de réjection des bruits additifs de lumière ambiante du circuit de préconditionnement 13.

La grille du transistor 157 est dans l'exemple décrit connectée au collecteur d'un transistor bipolaire 159 NPN dont la base est connectée par une résistance 160, par exemple une résistance comprise entre 5 Ω et 10 kΩ à la sortie de l'A.O. 135. L'émetteur du transistor bipolaire 159 est par exemple connecté à la masse. L'étage 132 peut encore comporter un interrupteur électronique non représenté, par exemple un transistor à effet de champ, pour interrompre le circuit de commande du transistor 157 lorsque la ou les LEDs du capteur 5 sont allumées, le condensateur 159 mémorisant alors la tension correspondant au photo-courant de lumière ambiante à compenser.

Un autre interrupteur analogique, par exemple un autre transistor MOS à canal P shuntant le condensateur 159 peut permettre sa décharge juste après l'extinction des LEDs infrarouge avant sa recharge pendant la durée d'extinction de ces LEDs à la tension correspondant à la compensation du photocourant de lumière ambiante.

Le microcontrôleur 15 exécute un algorithme agencé pour éliminer les artefacts rapides contenus dans le signal issu du circuit électronique de préconditionnement 13.

Le principe de base de cet algorithme est l'élimination des fronts descendants surnuméraires dus aux artefacts de mouvements rapides présents dans le signal logique issu de l'étage trigger flottant 136, par mesure de l'intervalle inter-battements et adaptation du seuil de discrimination battement/artefact.

On va maintenant décrire plus en détail un exemple d'algorithme de débruitage des artefacts rapides exécuté par le microcontrôleur 15, cet algorithme étant agencé pour comptabiliser le nombre de battements émis par intervalle de trente secondes.

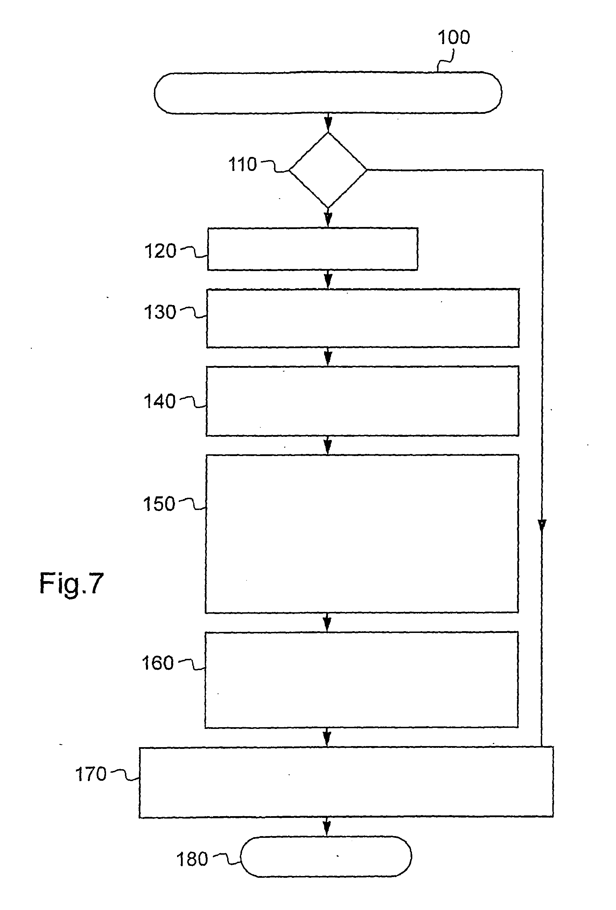

Le procédé de débruitage représenté à la figure 7 permet d'améliorer l'heuristique reposant sur la mesure de l'intervalle inter-battements grâce à une suppression forfaitaire de l'effet de bord, c'est-à-dire la soustraction d'un nombre de battements indépendants de la durée de la zone bruitée, aux extrémités des zones subissant une perturbation ininterrompue par des artefacts rapides détectés par l'algorithme, de manière à centrer l'erreur résiduelle.

Cette figure 7 représente un exemple du sous-programme d'interruption exécuté par le microcontrôleur 15. Ce sous-programme est sollicité périodiquement, par exemple toutes les quinze secondes, par le microcontrôleur 14 gérant les données issues des capteurs d'actimétrie 8, 9 et 11.

Lors d'une première étape 100, des données sont transmises par une liaison, par exemple une liaison série, provenant du microcontrôleur 14 au microcontrôleur 15 toutes les quinze secondes.

Lors d'une étape 110, le microcontrôleur 15 évalue si un signal d'appel d'urgence provenant du bouton d'appel 17 a été émis. Lorsque c'est le cas, le microcontrôleur exécute directement une étape 170 correspondant à la mise à « 1 » du drapeau déclenchant l'envoi radiofréquence de données par l'émetteur 20 à la base locale 30.

Lorsqu'aucun appel d'urgence n'a été émis, le microcontrôleur incrémente, au cours d'une étape 120, le compteur de battements ou la valeur du temps bruité courant.

A la suite de cette étape d'incrémentation, au cours d'une étape 130, une soustraction forfaitaire de par exemple un demi-battement par zone bruitée est effectuée, une zone bruitée de manière ininterrompue correspondant à une perturbation. Cette soustraction permet de prendre en compte l'effet de bord et de centrer l'erreur de mesure résiduelle. Au cours de cette étape, la valeur de pouls est corrigée une première fois selon l'équation :

$$\text{pouls }(n) = \text{pouls }(n) - \frac{\text{nombre de zones de bruit}}{2}.$$

Au cours d'une étape 140, le microcontrôleur 15 effectue une addition, prorata temporis du temps bruité, d'un nombre de battements correspondant au pouls corrigé à l'instant précédent et disponible en sortie de l'algorithme. Cette étape peut permettre de combler la durée cumulée des zones bruitées pendant la période courante de comptage, par exemple de quinze secondes, selon la relation suivante :

$$\text{pouls }(n) = \text{pouls }(n) + \text{temps bruité} \times \text{pouls }(n - 1)$$

Au cours de l'étape 150, le pouls est à nouveau corrigé. Au cours de cette étape 150, les deux derniers intervalles (n) et (n-1) sont considérés. Plusieurs cas peuvent alors se présenter:

- si les deux derniers intervalles de quinze secondes présentent chacun un taux temporel de bruitage supérieur à 17 %, l'algorithme provoque l'envoi d'une alarme d'échec de débruitage et procède à une réduction du seuil temporel de discrimination battement/artefact, le nouveau seuil ne pouvant être inférieur au seuil initialement fixé à 0,3 seconde. On parle ici d'échec du débruitage.
- si seul l'un des deux derniers intervalles de quinze secondes présente un taux temporel de bruitage supérieur à 17 %, c'est-à-dire si

$$\text{temps bruité }(n) > 17\ \%\ \times\ 15s$$

ou

$$\text{temps bruité }(n\text{-}1) > 17\ \%\ \times\ 15s$$

alors le pouls sur 30 secondes est calculé sur l'intervalle de quinze secondes ne présentant pas plus de 17 % de temps bruité,
- si le taux temporel de bruitage durant les dernières trente secondes est inférieur à 17 %, et supérieur à 5 %, l'algorithme calcule une moyenne sur les deux derniers intervalles de quinze secondes, pondérée par les durées bruitées de chaque intervalle selon la relation suivante :

$$\text{pouls/30s} = \text{pouls (n)} + \text{pouls (n-1)} + [\text{pouls (n-1)} - \text{pouls (n)}] \times \frac{\text{temps bruité (n)} - \text{temps bruité (n-1)}}{\text{temps bruité (n)} + \text{temps bruité (n-1)}}$$

- si le taux temporel de bruitage durant les trente dernières secondes est inférieur à 5 %, l'algorithme calcule le pouls sur ces trente secondes selon la relation suivante :

$$\text{pouls/30s} = \text{pouls (n)} + \text{pouls (n} - 1)$$

L'étape 160 correspond à une réduction du seuil de discrimination battement/artefact.

Deux cas peuvent alors se présenter :

- en cas d'échec du débruitage à l'étape 150, c'est-à-dire lorsque les deux derniers intervalles de quinze secondes présentent chacun un taux temporel de bruitage supérieur à 17 %, le nouveau seuil discriminant est choisi comme étant égal à une fraction, par exemple à trois quarts, du seuil de discrimination précédent,
- dans les autres cas, un estimateur récursif du seuil de discrimination est utilisé. Cet estimateur récursif permet une amélioration sensible de la suppression des fronts d'artefacts rapides en adaptant le seuil de discrimination battement/artefact initial de 0,3 seconde au pouls moyen du patient.

Un indice de convergence de cet estimateur permet ensuite d'effacer la suppression heuristique de l'effet de bord effectuée à l'étape 130 qui n'a plus lieu d'être si l'estimateur converge, c'est-à-dire si le pouls du patient ne s'écarte pas sensiblement de son pouls moyen.

La figure 8 représente un exemple de procédé d'estimation récursive du seuil de discrimination battement/ artefact. Ce procédé a pour entrée l'inverse du pouls sur les trente dernières secondes multiplié par un facteur de 0,6 pour assurer la détection de tout artefact placé entre deux battements réels consécutifs tout en ménageant une possibilité d'augmentation de la fréquence cardiaque du patient jusqu'à par exemple 1, 67 fois le pouls moyen courant. La valeur résultante est ensuite multipliée par une constante $\alpha$ très inférieure à 1. Cette nouvelle valeur est ensuite additionnée au seuil de discrimination précédent retardé de la période d'échantillonnage et multiplié par (1 - $\alpha$). $\alpha$ est par exemple compris entre 1/100 et 1/2. Dans l'exemple décrit, $\alpha$ est égal à 1/30, ce qui confère une durée de convergence de l'estimateur récursif comprise entre 30 min et 45 min.

Si le seuil de discrimination battements/artefact élaboré par l'estimateur récursif représenté à la figure 8 est supérieur ou égal à la moitié de l'interbattement moyen calculé sur les trente dernières secondes, la suppression forfaitaire de l'effet de bord effectuée à l'étape 130 n'a plus lieu d'être, tout battement résultant d'un artefact s'insérant entre deux battements réels consécutifs étant alors soit détecté par l'algorithme comme artefact et n'incrémente pas le compteur de battements réels à l'étape 120, soit détecté comme battement réel, ce qui entraîne la fausse interprétation du battement réel suivant comme artefact par l'algorithme, ces deux cas ayant la même incidence sur le cumul des battements réels durant un intervalle de quinze secondes.

A la suite de ce procédé d'estimation récursive du seuil de discrimination battement/artefact, le microcontrôleur 15 exécute l'étape 170 qui déclenche l'envoi radiofréquence par l'émetteur 20 d'informations à la base locale 30, comme par exemple le nombre de battements au cours des trente dernières secondes.

L'étape 180 constitue un retour d'interruption.

Le microcontrôleur 33 qui gère le récepteur de la base locale 30 peut contrôler par divers tests l'intégrité du signal reçu afin d'empêcher que des signaux radio non destinés à la base domestique ne puissent être interprétés par celle-ci comme des informations issues du terminal multicapteur 1.

L'invention n'est pas limitée aux exemples de mise en oeuvre qui viennent d'être décrits.

Le terminal multicapteur peut par exemple être couplé à un dispositif domotique de surveillance du patient, comme par exemple des capteurs infrarouges répartis à différents endroits des pièces du domicile du patient et permettant la localisation du patient dans son domicile, ou par exemple encore être couplé à un sol actimétrique localisant le patient dans son domicile.

En variante, le dispositif domotique de surveillance du patient auquel peut être couplé le terminal multicapteur peut comporter un réseau de récepteurs/émetteurs selon la norme Zigbee permettant de localiser le patient tout en sécurisant les liaisons entre son boîtier multicapteur et la base locale, les émetteurs/récepteurs communiquant par exemple avec la base locale par courants porteurs.

L'expression « comportant un » doit être comprise comme étant synonyme de « comportant au moins un ».

## Revendications

1. Dispositif (1) de mesure du pouls comportant :

    - un capteur de pouls (5) de type photopléthysmographique, de préférence porté à l'oreille, comportant au moins une source lumineuse, notamment une source de lumière infrarouge, et un composant (130) sensible à la lumière émise par la source, notamment un unique composant, et
    - un système de débruitage,
    le système de débruitage comportant :

        - un circuit électronique de préconditionnement (13) agencé pour éliminer les artefacts lents d'un signal représentatif du pouls acquis par le composant sensible à la lumière, le circuit électronique de préconditionnement (13) comportant une chaîne de filtrage (131) du signal capté, la chaîne de filtrage (131) comportant un étage (133) de filtrage en peigne configuré pour éliminer les fréquences multiples du fondamental de l'éclairage électrique alimenté par le secteur et,
        - au moins un microcontrôleur agencé pour traiter le signal délivré par le circuit électronique de préconditionnement et éliminer les artefacts rapides.

2. Dispositif selon la revendication 1, la source lumineuse étant alimentée par un courant discontinu de rapport cyclique inférieur à 1/10, notamment compris entre 1/40 et 1/10.

3. Dispositif selon l'une des revendications précédentes, la chaîne de filtrage (131) comportant un étage (132) de soustraction des bruits additifs.

4. Dispositif selon la revendication 3, l'étage (133) de filtrage comportant deux échantillonneur-bloqueurs (140, 141) commandés en alternance.

5. Dispositif selon l'une des revendications 3 et 4, la chaîne de filtrage (131) comportant un filtre coupant les fréquences à l'extérieur d'une fenêtre spectrale comprise entre 0.5 Hz et 20 Hz, notamment entre 0.5 Hz et 10 Hz.

6. Dispositif selon l'une quelconque des revendications 3 à 5, le circuit électronique de préconditionnement (13) comportant un comparateur de tension à hystérésis (136) comparant un signal issu de la chaîne de filtrage et une version retardée de ce même signal.

7. Dispositif selon l'une quelconque des revendications précédentes, le circuit électronique de préconditionnement (13) étant dépourvu de convertisseur analogique-numérique.

8. Système de surveillance mobile d'un patient, comportant :

    - un terminal multicapteur (1) comportant un boîtier (2) à porter par le patient, le terminal incluant le dispositif de mesure du pouls selon l'une quelconque des revendications précédentes et,
    - une base locale de traitement (30) pour recevoir et traiter des informations envoyées, notamment selon une période prédéfinie, par le terminal multicapteur (1).

9. Système selon la revendication 8, le terminal multicapteur comportant des moyens de fixation à une ceinture.

10. Système selon l'une des revendications 8 et 9, le terminal multicapteur (1) comportant au moins un capteur d'actimétrie choisi parmi : un capteur de mouvement isotrope (11), un capteur d'inclinaison (8) et un capteur d'impact de chute (9).

11. Système selon la revendication 10, le terminal multicapteur comportant le capteur d'impact de chute (9), comportant quatre branches, chaque branche comportant en série un capteur d'inclinaison et un capteur d'accélération.

**12.** Procédé pour mesurer le pouls d'une personne au moyen d'un dispositif comportant un capteur de pouls (5) comportant au moins une source lumineuse et un composant (130) sensible à la lumière émise par la source, notamment un unique composant, et un système de débruitage, le système de débruitage utilisé comportant :

- un circuit électronique de préconditionnement (13) agencé pour éliminer les artefacts lents d'un signal représentatif du pouls acquis par le composant (130) sensible à la lumière, le circuit électronique de préconditionnement (13) comportant une chaîne de filtrage (131) du signal capté, la chaîne de filtrage (131) comportant un étage (133) de filtrage en peigne sur des fréquences multiples du fondamental de l'éclairage électrique, alimenté par le secteur, et
- au moins un microcontrôleur agencé pour traiter le signal délivré par le circuit électronique de préconditionnement et éliminer les artefacts rapides.

**13.** Procédé selon la revendication précédente, comportant une étape de prétraitement par le circuit électronique (13) de préconditionnement au cours de laquelle,

- le signal issu du capteur de pouls attaque un étage de soustraction des composantes additives du bruit à variations lentes contenues dans le signal,
- le signal est traité par un étage de filtrage passe-bas à temps de propagation de groupe constant et à suppression des fréquences multiples du fondamental de l'alimentation électrique, et
- le signal est mis sous forme logique au moyen d'un étage comparateur.

**14.** Procédé selon l'une quelconque des revendications 12 et 13, comportant une étape de post-traitement algorithmique par le microcontrôleur (15) au cours de laquelle ;

- une suppression de l'effet de bord est effectuée en soustrayant du nombre de battements mesurés par intervalle de temps un nombre prédéfini de battements par zone bruitée, notamment un demi-battement par zone bruitée,
- on ajoute à la valeur obtenue le nombre de battements corrigés lors de l'intervalle de temps précédent *prorata-temporis* du temps bruité,
- on compare le taux temporel de bruitage à une valeur de référence, et
- on adapte le seuil de discrimination battement/artefact.

**15.** Procédé selon la revendication 14, comportant une étape d'estimation récursive du seuil de discrimination battement/artefact.

**Claims**

**1.** Device (1) for measuring the pulse comprising:

- a pulse sensor (5) of photoplethysmographic type, preferably worn in the ear, comprising at least one light source, especially an infrared light source, and a component (130) sensitive to the light emitted by the source, especially a single component, and
- a denoising system,

the denoising system comprising:

- an electronic preconditioning circuit (13) configured to eliminate the slow artifacts of a signal representative of the pulse acquired by the light sensitive component, the electronic preconditioning circuit (13) comprising a filtering chain (131) of the sensed signal, the filtering chain (131) composing a comb filtering stage (133) configured to eliminate frequencies that are multiples of the frequency of the fundamental of the mains powered electrical lighting, and,
- at least one microcontroller configured to process the signal delivered by the electronic preconditioning circuit and eliminate the fast artifacts.

**2.** Device according to claim 1, the light source being supplied by a discontinuous current with duty ratio of less than 1/10, especially of between 1/40 and 1/10.

3. Device according to any one of the preceding claims, the filtering chain (131) comprising a stage (132) for subtracting additive noises.

4. Device according to claim 3, the filtering stage (133) comprising two sample-and-hold units (140, 141) controlled alternately.

5. Device according to one of claims 3 and 4, the filtering chain (131) comprising a filter cutting off the frequencies outside a spectral window of between 0.5 Hz and 20 Hz, especially between 0.5 Hz and 10 Hz.

6. Device according to any one of claims 3 to 5, the electronic preconditioning circuit (13) comprising a voltage comparator with hysteresis (136) comparing a signal arising from the filtering chain and a delayed version of this same signal.

7. Device according to any one of the preceding the electronic preconditioning circuit (13) being devoid of analog-digital converter.

8. Mobile monitoring system for a patient, comprising:

   - a multisensor terminal (1) comprising a box (2) to be worn by the patient, the terminal including the device for measuring the pulse as claimed in any one of the preceding claims and,
   - a local processing base (30) for receiving and processing information sent, especially according to a predefined period, by the multisensor terminal (1).

9. System according to claim 8, the multisensor terminal comprising means for fixing to a belt.

10. System according to any one of claims 8 and 9, the multisensor terminal (1) comprising at least one actimetry sensor chosen from among: an isotropic movement sensor (11), an inclination sensor (8) and a fall impact sensor (9).

11. System according to claim 10, the multisensor terminal comprising the fall impact sensor (9), comprising four arms, each arm comprising in series an inclination sensor and an acceleration sensor.

12. Method for measuring the pulse of a person by means of a device comprising a pulse sensor (5) comprising at least one light source and a component (130) sensitive to the light emitted by the source, especially a single component, and denoising system,
the used denoising system comprising:

   - an electronic preconditioning circuit (13) configured to eliminate the slow artifacts of a signal representative of the pulse acquired by the light sensitive component (130), the electronic preconditioning circuit (13) comprising a filtering chain (131) of the sensed signal, the filtering chain (131) comprising a stage (133) of comb filtering on frequencies that are multiples of the fundamental of the mains powered electrical lighting, and
   - at least one microcontroller configured to process the signal delivered by the electronic preconditioning circuit and eliminate the fast artifacts.

13. Method according to the preceding claim, comprising a step of preprocessing by the electronic preconditioning circuit (13) in the course of which,

   - the signal arising from the pulse sensor drives a stage for subtracting the additive components of the noise with slow variations contained in the signal,
   - the signal is processed by a low-pass filtering stage with constant group propagation time and an with removal of the frequencies which are multiples of the fundamental of the electrical power supply, and
   - the signal is put into logic form by means of a comparator stage.

14. Method according to any one of claims 12 and 13, comprising a step of algorithmic post-processing by the microcontroller (15) in the course of which:

   - a removal of the effect is performed by subtracting from the number of beats measured per time interval a predefined number of beats per noise-affected zone, especially a half-beat per noise-affected zone,
   - the number of beats corrected during the previous time interval *prorata-temporis* of the noise-affected time is added to the value obtained,
- the temporal noising rate is compared with a reference value, and
- the beat/artifact discrimination threshold is adapted.

15. Method according to claim 14, comprising a recursive step of estimating the beat/artifact discrimination threshold.


**Patentansprüche**

1. Vorrichtung (1) zur Pulsmessung umfassend:

   - einen Pulssensor (5) in photoplethysmografischer Ausführung, vorzugsweise am Ohr getragen, umfassend mindestens eine Lichtquelle, insbesondere eine Infrarotlicht-Quelle, und ein Bauteil (130), das gegenüber dem Licht empfindlich ist, das von der Quelle abgegeben wird, insbesondere ein einziges Bauteil, und
   - ein Entrauschungssystem,

   wobei das Entrauschungssystem Folgendes umfasst:

   - ein elektronischer Vorkonditionierungsschaltkreis (13), der so aufgebaut ist, dass die langsamen Artefakte eines für den Puls repräsentativen Signals, das von dem lichtempfindlichen Bauteil erfasst wird, beseitigt werden, wobei der elektronische Vorkonditionierungsschaltkreis (13) eine Filterungskette (13 1) für das erfasste Signal umfasst, wobei die Filterungskette eine Filterungsstufe (133) in Kammform umfasst, welche so konfiguriert ist, dass die Frequenzen beseitigt werden, die ein Mehrfaches der Grundfrequenz der vom Netz gespeisten elektrischen Beleuchtung sind, und
   - mindestens einen Mikrocontroller, der so aufgebaut ist, dass das von dem elektronischen Vorkonditionierungsschaltkreis gelieferte Signal verarbeitet wird und die schnellen Artefakte beseitigt werden.

2. Vorrichtung gemäß Anspruch 1, wobei die Lichtquelle von einem pulsierenden Strom mit einem zyklischen Verhältnis von kleiner als 1:10 versorgt wird, das insbesondere zwischen 1:40 und 1:10 liegt.

3. Vorrichtung gemäß einem der vorstehenden Ansprüche, wobei die Filterungskette (131) eine Stufe (132) zum Abziehen des zusätzlichen Rauschens umfasst.

4. Vorrichtung Anspruch 3, wobei die Filterstufe (133) zwei Abtast- und Halteschaltungen (140, 141) umfasst, die im Wechsel angesteuert werden.

5. Vorrichtung gemäß einem der Ansprüche 3 und 4, wobei die Filterungskette (131) einen Filter umfasst, der die Frequenzen außerhalb eines Spektralfensters zwischen 0,5 Hz und 20 Hz, insbesondere zwischen 0,5 Hz und 10 Hz, abschneidet.

6. Vorrichtung gemäß einem beliebigen der Ansprüche 3 bis 5, wobei der elektronische Vorkonditionierungsschaltkreis (13) einen Hysterese-Spannungsvergleicher (136) umfasst, der ein aus der Filterungskette hervorgegangenes Signal und eine zeitverzögerte Version desselben Signals vergleicht.

7. Vorrichtung gemäß einem beliebigen der vorstehenden Ansprüche, wobei der elektronische Vorkonditionierungsschaltkreis (13) nicht mit einem Analog-Digital-Wandler ausgestattet ist.

8. Mobiles Patientenüberwachungssystem, umfassend:

   - ein Mehrsensorterminal (1) mit einem vom Patienten zu tragenden Gehäuse (2), wobei das Terminal die Pulsmessvorrichtung gemäß einem beliebigen der vorstehend genannten Ansprüche beeinhaltet, und
   - eine lokale Verarbeitungsbasis (30) zum Empfangen und Verarbeiten der Daten, die vom Mehrsensorterminal (1) insbesondere gemäß einem im Vorhinein festgelegten Intervall geschickt werden.

9. System gemäß Anspruch 8, wobei das Mehrsensorterminal Mittel zum Befestigen an einem Gürtel umfasst.

10. System gemäß einem der Ansprüche 8 und 9, wobei das Mehrsensorterminal (1) mindestens einen Aktimetrie-Sensor umfasst, für den einer der folgenden Typen gewählt wird: ein isotroper Bewegungssensor (11), ein Neigungssensor (8) und ein Sturz-Aufschlag-Sensor (9).

11. System gemäß Anspruch 10, wobei das Mehrsensorterminal den Sturz-Aufschlag-Sensor (9) umfasst, welcher vier Zweige umfasst, wobei jeder Zweig in Serie einen Neigungssensor und eine Beschleunigungssensor umfasst.

12. Verfahren zur Messung des Pulses einer Person mit Hilfe einer Vorrichtung, die einen Pulssensor (5) umfasst, welcher mindestens eine Lichtquelle und ein Bauteil (130) umfasst, die für das von der Quelle abgegebene Licht empfindlich ist, insbesondere ein einziges Bauteil, und ein Entrauschungssystem, wobei das verwendete Entrauschungssystem Folgendes umfasst:

- ein elektronischer Vorkonditionierungsschaltkreis (13), der so aufgebaut ist, dass die langsamen Artefakte eines für den Puls repräsentativen Signals, das von dem lichtempfindlichen Bauteil (130) erfasst wird, beseitigt werden, wobei der elektronische Vorkonditionierungsschaltkreis (13) eine Filterungskette (131) für das erfasste Signal umfasst, wobei die Filterungskette (131) eine Filterungsstufe (133) in Kammform für die Frequenzen umfasst, die ein Mehrfaches der Grundfrequenz der vom Netz gespeisten elektrischen Beleuchtung sind, und
- mindestens einen Mikrocontroller, der so aufgebaut ist, dass das von dem elektronischen Vorkonditionierungsschaltkreis gelieferte Signal verarbeitet wird und die schnellen Artefakte beseitigt werden,

13. Verfahren gemäß dem vorstehenden Anspruch, umfassend eine Etappe der Vorverarbeitung durch den elektronischen Vorkonditionierungsschaltkreis (13), während der

- das aus dem Pulssensor hervorgegangene Signal eine Stufe angreift, die die zusätzlichen Rauschkomponenten mit langsamen Veränderungen abzieht, welche im Signal enthalten sind,
- das Signal von einer Tiefpassfilter-Stufe mit konstanter Gruppenausbreitungszeit und Unterdrückung der Mehrfachfrequenzen der Grundfrequenz der Stromversorgung verarbeitet wird, und
- das Signal mit Hilfe einer Vergleicherstufe in logische Form umgewandelt wird.

14. Verfahren gemäß einem der Ansprüche 12 und 13, umfassend eine Phase der algorithmischen Nachverarbeitung durch Mikrocontroller (15), während der:

- eine Unterdrückung des Randeffekts erfolgt, indem von der Anzahl der pro Zeitintervall gemessenen Pulsschläge eine im Vorhinein festgelegte Anzahl von Pulsschlägen pro Rauschbereich abgezogen wird, insbesondere ein halber Pulsschlag pro Rauschbereich,
- zu dem resultierenden Wert die Anzahl von Pulsschlägen hinzugezählt wird, die im vorhergehenden Intervall zeitanteilig um die Rauschzeit korrigiert wurden,
- die zeitliche Rauschrate mit einem Sollwert verglichen wird, und
- die Pulsschlag/Artefakt-Diskriminierungsschwelle angepasst wird.

15. Verfahren gemäß Anspruch 14, umfassend eine Phase der rekursiven Schätzung der Pulsschlag/Artefakt-Diskri-minierungsschwelle.

Fig.1

Fig.2

Fig.3

Fig.4

## Fig.5

## Fig.6

## Fig.11

Fig.7

## Fig.8

$(pouls/30s)^{-1} \times 0,6$ → α → + → Seuil de discrimination

1-α ← Retard Te

## Fig.9

32 → 33 → 35 → 37

## Fig.10

18

22 23

18b

24 25

18a

19

**EP 2 122 596 B1**

## RÉFÉRENCES CITÉES DANS LA DESCRIPTION

**Documents brevets cités dans la description**

- FR 2829862
- US 7175601 B
- EP 1297784 A
- WO 9111956 A